# NOUVEAU FASCICULE DE BREVET EUROPEEN

(11) **EP 1 648 860 B2**
(45) Date de publication et mention de la décision concernant l'opposition: **10.09.2014**
(45) Mention de la délivrance du brevet: 28.12.2011
(21) Numéro de dépôt: 04767766.1
(22) Date de dépôt: 23.07.2004
(51) Int. Cl.: C07C 253/34, C07C 253/10, C07C 255/04

(54) **PROCEDE DE FABRICATION ET DE SEPARATION DE COMPOSES DINITRILES**
VERFAHREN ZUR HERSTELLUNG UND TRENNUNG VON DINITRILEN
METHOD OF PRODUCING AND SEPARATING DINITRILE COMPOUNDS

(30) Priorité: 25.07.2003 FR 0309152
(43) Date de publication de la demande: 26.04.2006
(73) Titulaire: Invista Technologies S.à.r.l., 9000 St. Gallen (CH)
(72) Inventeur: GERBER, Jérôme, F-69008 LYON (FR); LECONTE, Philippe, F-69330 MEYZIEU (FR); AMOROS, Daniel, F-69200 Venissieux (FR)
(74) Mandataire: Carpmaels & Ransford LLP
(86) Numéro de dépôt international: PCT/FR2004/001972
(87) Numéro de publication internationale: WO 2005/019160

(56) Documents cités:
- DE-A1- 19 832 529
- DE-B- 1 268 611
- DE-B- 1 268 611
- FALBE, JÜRGEN, REGITZ, MANFRED: 'Römpp Chemie Lexikon', vol. 9, 2000, GEORG THIEME VERLAG, STUTTGART Bd. 'Adipinsäuredinitril', pages 56 - 57
- YEN-CHEN, YEN AND SHIH-YUEH,WU: 'Process Economics SRI International', vol. 54B, Septembre 1987, PROCESS ECONOMICS PROGRAM, MENLO PARK, CALIFORNIA 94025 Bd. 'Nlon 66', pages 1 - 35
- PROCESS ECONOMICS PROGRAM SRI INTERNATIONAL: 'Process Economics Report No. 31B', Mars 1997
- JAMES M. DOUGLAS: 'Conceptual Design of Chemical Processes', 1988, MCGRAW-HILL, ISBN 0-070127762-7 pages 163,180, 182 - 184
- K. WEISSERMEL, H.-J. ARPE: 'Industrielle Organische Chemie', 1988, VCH VERLAGSGESELLSCHAFT MBH, WEINHEIM; BASEL (SCHWEIZ), ISBN 3-527-26731-X pages 261 - 264
- M.GUISNET ET AL.: 'Heterogeneous Catalysis and Fine Chemicals III, Proceedings of the 3rd International Symposium, Poitiers, April 5-8, 1993, vol. 78, pages 291-298' ELSEVIER SCIENCE vol. 78, 1993, pages 291 - 298
- PATRICIA PÉREZ, ALEXANDRO TORO-LABBÉ: 'Theoretical analysis of some substituted imine-enamine tautomerism' THEORETICAL CHEMISTRY ACCOUNTS vol. 105, 19 Janvier 2001, pages 422 - 430 DOI: 10.1007/s002140000223

## Description

La présente invention concerne un procédé de fabrication et de séparation de composés dinitriles.

Elle se rapporte, plus particulièrement, à un procédé de fabrication et de séparation de composés dinitriles à partir d'un milieu provenant de l'hydrocyanation de mononitriles insaturés.

Les composés dinitriles tels que l'adiponitrile sont des intermédiaires chimiques importants pour la fabrication de nombreux composés. L'adiponitrile est, notamment, un intermédiaire chimique pour la synthèse de différents monomères utilisés dans la fabrication de polymère tels que les polyamides.

Par ailleurs, le procédé de synthèse de composés dinitriles utilisé industriellement consiste à réaliser une hydrocyanation en deux étapes de dioléfines, par exemple du butadiène.

Dans une première étape, la synthèse permet de produire des mononitriles insaturés. Ces derniers sont utilisés comme matière première dans une seconde étape pour être transformés en dinitriles par réaction avec une molécule de cyanure d'hydrogène. Généralement, ces deux étapes sont réalisées successivement avec une circulation des différents flux, comme par exemple le flux de système catalytique, entre celles-ci. Toutefois, elles peuvent être conduites de manière séparée et indépendante.

Le milieu réactionnel issu de la deuxième étape contient les dinitriles formés, les mononitriles non transformés et le système catalytique, ainsi que des sous-produits divers.

Les procédés comprennent des étapes de traitement de ce milieu réactionnel pour d'une part séparer le système catalytique et d'autre part séparer les dinitriles, des mononitriles qui seront avantageusement recyclés.

La séparation du système catalytique est généralement obtenue par décantation et/ou par extraction liquide/liquide avec un solvant d'extraction tels que des hydrocarbures. La phase organique comprenant les mononitriles et dinitriles est traitée dans une étape de distillation pour séparer les mononitriles des dinitriles d'une part et les produits lourds d'autre part voir par exemple DE 1268611B. Cette étape de distillation comprend, dans les procédés actuels, deux colonnes de distillation, dans une première colonne les produits de point d'ébullition plus bas que celui des dinitriles, tels que les mononitriles sont séparés et récupérés en tête de colonne, la fraction lourde contenant les dinitriles est alimentée dans une seconde colonne de distillation permettant de distiller les dinitriles qui sont récupérés en tête de colonne.

Ce procédé présente plusieurs inconvénients dont celui de maintenir les composés dinitriles à une température élevée pendant une durée relativement longue.

Ce maintien à une température élevée favorise la formation de sous produits provenant de la dégradation des dinitriles. Ainsi dans le cas de l'adiponitrile, il se forme notamment un sous produit, l'iminocyanocyclopentane (ICCP). Ce sous produit est très difficilement séparable de l'adiponitrile. En outre, dans les procédés de transformation de l'adiponitrile en d'autres composés comme par exemple, en hexaméthylène diamine, les sous produits peuvent être transformés en d'autres composés difficilement séparables. Ainsi, au cours de l'hydrogénation de l'adiponitrile en hexaméthylènediamine, l'ICCP est transformé en aminométhylènecyclopentaneamine (AMCPA). Ces impuretés ne peuvent être admises dans les procédés de fabrication de polymères tels que le polyamide, notamment quand ceux-ci sont utilisés pour la fabrication de fils textiles.

Un des buts de la présente invention est de remédier à ces inconvénients en proposant un nouveau procédé de préparation et de séparation de composés dinitriles permettant, notamment, de limiter et minimiser la formation de sous-produits pendant la récupération et extraction des composés dinitriles formés.

La présente invention a pour objet un procédé de fabrication et de séparation de dinitriles à partir d'un milieu provenant de l'hydrocyanation de mononitriles insaturés caractérisé en ce qu'il consiste à
➢ Alimenter le milieu contenant les composés dinitriles et les mononitriles insaturés dans une colonne de distillation, avantageusement au niveau d'un plateau théorique de la colonne
➢ A récupérer en tête de colonne les composés de point d'ébullition plus bas que celui des dinitriles comprenant les mononitriles insaturés présents dans le milieu
➢ A récupérer la fraction intermédiaire contenant les dinitriles à partir d'un plateau théorique situé à une partie inférieure de la colonne par rapport au plateau d'alimentation du milieu contenant les dinitriles
➢ A récupérer les produits de point d'ébullition plus élevé que celui des dinitriles en pied de colonne.

Selon une caractéristique de l'invention, la température de pied de colonne est inférieure à 200°C, de préférence comprise entre 140°C et 190°C. Une telle température de pied de colonne permet de limiter la formation de sous-produits notamment par dégradation thermique des dinitriles. Par température de pied de colonne, on entend la température de la phase liquide dans le bouilleur de la colonne ainsi que la température de paroi dudit bouilleur.

Avantageusement, la fraction intermédiaire comprenant les dinitriles est récupérée sans reflux ou avec un reflux. Le taux de reflux peut représenter de 1 à 6 % en poids de la fraction récupérée. Le reflux peut être introduit dans la colonne au même niveau que le prélèvement de la dite fraction ou à un niveau différent.

Selon une autre caractéristique de l'invention, les composés dinitriles produits par le procédé de l'invention sont des composés de formule générale (I) suivante.

NC - R -CN (I)

dans laquelle le radical R représente un radical hydrocarboné saturé comprenant de 2 à 10 atomes de carbone.

Les composés dinitriles préférés de l'invention sont choisis dans le groupe comprenant l'adiponitrile, le méthyl-glutaronitrile, l'éthyl succinonitrile.

La distillation est réalisée sous une pression adaptée en fonction de la nature des composés dinitriles à séparer, de préférence à une pression comprise entre 1 kPa et 5 kPa.

La distillation de l'invention peut être réalisée dans tout dispositif convenable tel que colonne à plateaux, colonne à remplissage, colonne à cloisons. Le nombre de plateaux théoriques de la colonne est déterminé en fonction de la nature des composés à traiter. Généralement, des colonnes présentant un nombre de plateaux théoriques compris entre 6 et 20 sont convenables pour l'invention.

La fraction intermédiaire comprenant les composés dinitriles est soutirée de la colonne de distillation avantageusement à l'état liquide ou à l'état vapeur. La fraction recueillie en tête de colonne contient, dans le cas d'un procédé d'hydrocyanation, les mononitriles insaturés présents dans le milieu.

Le procédé de l'invention permet de récupérer des dinitriles contenant une très faible quantité de sous-produits provenant de la décomposition thermique des composés dinitriles. Les dinitriles ainsi récupérés peuvent être soumis à une séparation, par exemple par distillation, pour récupérer d'une part l'adiponitrile et d'autre part les autres dinitriles formés comme le méthyl-glutaronitrile, l'éthyl succinonitrile.

Par ailleurs, le procédé de l'invention permet d'obtenir des composés dinitriles contenant une très faible concentration d'impuretés en utilisant une seule colonne de distillation, c'est à dire avec un coût d'investissement et de fonctionnement réduit par rapport aux installations actuelles comprenant deux colonnes montées en série.

D'autres avantages, détails de l'invention apparaîtront plus clairement au vu de la description d'un exemple de réalisation du procédé de l'invention donné uniquement à titre d'illustration et fait en référence aux figures annexées dans lesquelles :
- la figure 1 représente un schéma synoptique d'une installation de séparation de dinitriles de l'art antérieur,
- la figure 2 représente un schéma synoptique d'un mode de réalisation d'une installation de séparation de dinitriles conforme à l'invention.

### EXEMPLE COMPARATIF 1 :

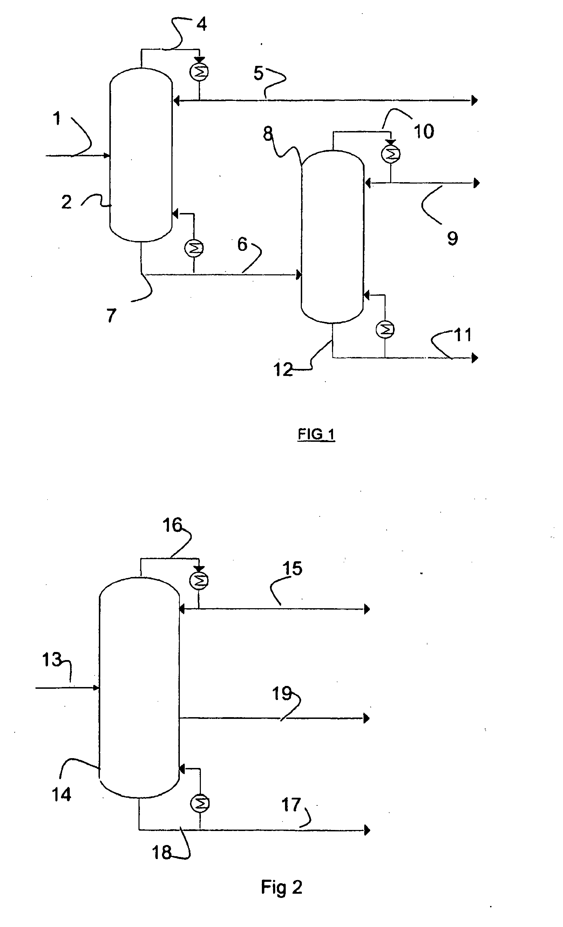

En référence à la figure 1, un mélange issu d'un procédé de fabrication d'adiponitrile par hydrocyanation de pentènenitriles en présence d'un système catalytique comprenant un complexe organométallique de nickel et de tritolylphosphite et d'un acide de Lewis (ZnCl₂), est alimenté en 1 dans une première colonne 2 de distillation comprenant un garnissage et présentant un nombre de plateaux théoriques égal à 6. La température de pied de colonne est de 161°C, et la pression de fonctionnement est de 2 kPa.

Le soutirage d'une fraction de tête 5 est réalisé avec un reflux 4. la fraction 5 soutirée comprend les produits présentant une température d'ébullition inférieur à celle de l'adiponitrile ou plus généralement à celles des dinitriles présents dans le milieu. Comme cela est indiqué dans le tableau I ci-dessous, cette fraction de tête est constituée principalement de pentènenitriles qui n'ont pas été transformés en dinitriles.

Le liquide présent en fond de colonne circule dans une boucle 7 comprenant un bouilleur. Une fraction 6 de queue est soutirée en fond de colonne 2 ou à partir de cette circulation.

Cette fraction 6 est alimentée dans une seconde colonne 8 de distillation. Dans l'exemple illustré, la colonne 8 est semblable à la colonne 2. La température de pied de colonne est de 164°C et la pression de 2 kPa.

L'alimentation de la fraction 6 est réalisée au niveau d'un plateau intermédiaire de la colonne 8. Les dinitriles dont l'adiponitrile sont récupérés sous forme de fraction de tête 9, avec un reflux 10. Les composés de point d'ébullition plus élevé que celui de l'adiponitrile et des dinitriles présents sont récupérés sous forme d'une fraction de queue 11. Comme dans la colonne 2, une circulation 12 du liquide présent dans le fond de colonne est réalisée avec présence d'un bouilleur dans la boucle de circulation.

La composition des différentes fractions est indiquée dans le tableau I ci-dessous.

Le temps de séjour du mélange et notamment de l'adiponitrile et des dinitriles est la somme des temps de séjour dans les colonnes 2 et 8.

La concentration en ICCP dans la fraction de tête 9 de la seconde colonne 8 est de 0,08 % en poids.

**Tableau I**

| | mélange1 | Fraction 5 | Fraction 6 | Fraction 9 | Fraction 11 |
|---|---|---|---|---|---|
| pentènenitriles (% poids) | 19,6 | 97,6 | 0,16 | 0,17 | 0 |
| Dinitriles (% poids) | 80 | 2,4 | 99,34 | 99,82 | 90,99 |
| composés lourds (% poids) | 0,4 | 0 | 0,5 | 0,01 | 9,01 |
| flux total (kmol/h) | 1 | 0,2 | 0,8 | 0,76 | 0,04 |

Par composés lourds, on désigne les composés de point d'ébullition supérieur à celui de l'adiponitrile

### EXEMPLE 2

En référence à la figure 2, un mélange correspondant à celui de l'exemple1 est alimenté en 13 sur un plateau intermédiaire d'une colonne 14 de distillation à garnissage. Cette colonne présente 6 plateaux théoriques et fonctionne sous une pression de 2 kPa avec une température de pied de colonne de 164°C.

Les composés de point d'ébullition plus faible que ceux des dinitriles présents, plus particulièrement de celui de l'adiponitrile sont récupérés en tête de colonne 14 avec un reflux 16. La composition de cette fraction de tête 15 est indiquée dans le tableau II ci-dessous et comprend notamment les pentènenitriles qui n'ont pas été transformés.

Les composés de point d'ébullition plus élevé que ceux des dinitriles présents, plus particulièrement de celui de l'adiponitrile sont récupérés en pied de colonne 14 sous forme d'une fraction 17 avec une circulation du liquide en fond de colonne à travers un boucle 18 comprenant un bouilleur. La fraction 17 peut être avantageusement concentrée avant d'être recyclée dans la boucle 18.

Les dinitriles dont l'adiponitrile sont récupérés dans une fraction 19 par soutirage à partir d'un plateau intermédiaire situé à une position inférieure par rapport à l'alimentation 13 du mélange à traiter.

La composition des différentes fractions est indiquée dans le tableau II ci-dessous.

Le temps de séjour du mélange et notamment des dinitriles dont l'adiponitrile est de même ordre de grandeur que celui observé dans la colonne 8 de l'exemple 1. Ainsi, le procédé de l'invention permet de séparer et récupérer les dinitriles dont l'adiponitrile avec un temps de séjour de ce composé dans la colonne de distillation inférieur à celui observé dans l'exemple 1. En effet, le temps de séjour dans la colonne 2 est supprimé avec le procédé de l'invention.

Le procédé de l'invention permet de récupérer des dinitriles contenant 0,04 % en poids d'ICCP.

**Tableau II**

| | Melange 1 | Fraction 15 | Fraction 19 | Fraction 17 |
|---|---|---|---|---|
| Pentènenitriles (% poids) | 19,6 | 97,6 | 0,17 | 0 |
| adiponitrile (% poids) | 80 | 2,4 | 99,82 | 90,99 |
| composés lourds (% poids) | 0,4 | 0 | 0,01 | 9,01 |
| flux total (kmol/h) | 1 | 0,2 | 0,76 | 0,04 |

## Revendications

1. Procédé de fabrication et de séparation de dinitriles à partir d'un milieu provenant de l'hydrocyanation de mononitriles insaturés **caractérisé en ce qu'**il consiste à
➢ Alimenter le milieu contenant les composés dinitriles et les mononitriles insaturés dans une colonne de distillation
➢ A récupérer en tête de colonne, les composés de point d'ébullition plus bas que celui des dinitriles comprenant les mononitriles insaturés présents dans le milieu
➢ A récupérer la fraction intermédiaire contenant les dinitriles à partir d'un plateau théorique situé à une partie inférieure de la colonne par rapport au point d'alimentation du milieu contenant les dinitriles, et
➢ A récupérer en pied de colonne, les produits de point d'ébullition plus élevé que celui des dinitriles.

2. Procédé selon la revendication1, **caractérisé en ce que** la température de pied de colonne est inférieure à 200°C, de préférence comprise entre 140°C et 190°C.

3. Procédé selon la revendication 1 ou 2, **caractérisé en ce que** la récupération de la fraction intermédiaire est récupérée sans reflux.

4. procédé selon la revendication 1 ou 2, **caractérisé en ce que** la récupération de la fraction intermédiaire est effectuée avec un taux de reflux compris entre 1 et 6% en poids de la fraction intermédiaire.

5. procédé selon l'une des revendications précédentes, **caractérisé en ce que** les composés dinitriles sont des composés de formule générale (I) suivante.
NC - R -CN (I)
dans laquelle le radical R représente un radical hydrocarboné saturé comprenant de 2 à 10 atomes de carbone.

6. Procédé selon la revendication 5, **caractérisé en ce que** les dinitriles sont choisis dans le groupe comprenant l'adiponitrile, le méthyl-glutaronitrile, l'éthyl succinonitrile.

7. Procédé selon l'une des revendications précédentes, **caractérisé en ce qu'**il est réalisé sous une pression comprise en 1 kPa et 5 kPa.

8. Procédé selon l'une des revendications précédentes, caractérisé en ce la colonne de distillation est une colonne à plateaux, une colonne à garnissage ou une colonne à cloisons.

## Patentansprüche

1. Verfahren zur Herstellung und Abtrennung von Dinitrilen, ausgehend von einem Medium, das aus der Hydrocyanierung von ungesättigten Mononitrilen stammt, **dadurch gekennzeichnet, dass** es aus den folgenden Schritten besteht:
- Einspeisen des Mediums, welches die Dinitrilverbindungen und die ungesättigten Mononitrile enthält, in eine Destillationskolonne
- Entnehmen der Verbindungen mit einem Siedepunkt, der niedriger als derjenige der Dinitrile ist, umfassend die im Medium vorhandenen ungesättigten Mononitrile, am Kopf der Kolonne
- Entnehmen der Mittelfraktion, welche die Dinitrile enthält, an einem theoretischen Boden, der sich in einem unteren Teil der Kolonne befindet, unter Bezugnahme auf die Stelle, an der das Medium, welches die Dinitrile enthält, eingespeist wird, und
- Entnehmen der Produkte mit einem Siedepunkt, der höher als derjenige der Dinitrile ist, am Sumpf der Kolonne.

2. Verfahren nach Anspruch 1, **dadurch gekennzeichnet, dass** die Temperatur am Sumpf der Kolonne weniger als 200 °C beträgt, wobei sie vorzugsweise im Bereich von 140 °C bis 190 °C liegt.

3. Verfahren nach Anspruch 1 oder 2, **dadurch gekennzeichnet, dass** die Entnahme der Mittelfaktion ohne Rückfluss erfolgt.

4. Verfahren nach Anspruch 1 oder 2, **dadurch gekennzeichnet, dass** die Entnahme der Mittelfraktion mit einer Rückflussquote im Bereich von 1 bis 6 Gewichts-% der Mittelfraktion erfolgt.

5. Verfahren nach einem der vorhergehenden Ansprüche, **dadurch gekennzeichnet, dass** es sich bei den Dinitrilverbindungen um Verbindungen nach der folgenden allgemeinen Formel (I)
NC - R -CN (I)
handelt,
wobei der Rest R für einen gesättigten Kohlenwasserstoffrest steht, der 2 bis 10 Kohlenstoffatome umfasst.

6. Verfahren nach Anspruch 5, **dadurch gekennzeichnet, dass** die Dinitrile aus der Gruppe ausgewählt sind, die Adiponitril, Methylglutaronitril, Ethylsuccinonitril umfasst.

7. Verfahren nach einem der vorhergehenden Ansprüche, **dadurch gekennzeichnet, dass** es unter einem Druck im Bereich von 1 kPa bis 5 kPa durchgeführt wird.

8. Verfahren nach einem der vorhergehenden Ansprüche, **dadurch gekennzeichnet, dass** es sich bei der Destillationskolonne um eine Kolonne mit Böden, eine Kolonne mit Einbauten oder eine Kolonne mit Scheidewänden handelt.

## Claims

1. Process for the manufacture and separation of dinitriles from a medium originating from the hydrocyanation of unsaturated mononitriles, **characterized in that** it consists in
► feeding the medium comprising the dinitrile compounds and the unsaturated mononitriles to a distillation column
► recovering, at the column top, the compounds with a lower boiling point than that of the dinitriles comprising the unsaturated mononitriles present in the medium
► recovering the intermediate fraction comprising the dinitriles from a theoretical plate situated in a lower part of the column with respect to the feed point of the medium comprising the dinitriles, and
► recovering, at the column bottom, the products with a higher boiling point than that of the dinitriles.

2. Process according to Claim 1, **characterized in that** the column bottom temperature is less than 200°C, preferably between 140°C and 190°C.

3. Process according to Claim 1 or 2, **characterized in that** the recovery of the intermediate fraction is carried out without reflux.

4. Process according to Claim 1 or 2, **characterized in that** the recovery of the intermediate fraction is carried out with a reflux ratio of between 1 and 6% by weight of the intermediate fraction.

5. Process according to one of the preceding claims, **characterized in that** the dinitrile compounds are compounds of following general formula (I):
NC - R -CN (I)
in which the R radical represents a saturated hydrocarbonaceous radical comprising from 2 to 10 carbon atoms.

6. Process according to Claim 5, **characterized in that** the dinitriles are chosen from the group consisting of adiponitrile, methylglutaronitrile and ethylsuccinonitrile.

7. Process according to one of the preceding claims, **characterized in that** it is carried out under a pressure of between 1 kPa and 5 kPa.

8. Process according to one of the preceding claims, **characterized in that** the distillation column is a plate column, a packed column or a partition column.
